# EUROPEAN PATENT SPECIFICATION

(11) **EP 1 799 276 B1**
(45) Date of publication and mention of the grant of the patent: **03.05.2017**
(21) Application number: 05792097.7
(22) Date of filing: 07.10.2005
(51) Int. Cl.: A61L 26/00

(54) **COMPOSITION FOR TREATING WOUNDS AND BURNS**
ZUSAMMENSETZUNG ZUR BEHANDLUNG VON WUNDEN UND VERBRENNUNGEN
COMPOSITION POUR LE TRAITEMENT DES PLAIES ET DES BRULURES

(30) Priority: 07.10.2004 US 616231 P
(43) Date of publication of application: 27.06.2007
(73) Proprietor: Ngen Pharmaceuticals N.V., Curaçao (AN)
(72) Inventor: LEKHRAM, Changoer, NL-3404 HL Ijsselstein (NL); VAN DEN BOSCH, Willem, Frederik, NL-2343 JX Oegstgeest (NL); MEIJERINK, Hendrik, Jan, Cornelis, B-1000 Brussel (BE)
(74) Representative: Nederlandsch Octrooibureau
(86) International application number: PCT/NL2005/000729
(87) International publication number: WO 2006/038803

(56) References cited:
- WO-A-03/063923
- WO-A1-00/19981
- GB-A- 2 402 882
- US-A- 4 317 814
- US-A- 4 507 285
- US-A- 6 017 515
- 'Open wounds' INTERNET CITATION, [Online] Retrieved from the Internet: <URL:http://medical-dictionary.thefreedicti onary.com/open+wound>
- 'skin lesions' INTERNET CITATION, [Online] Retrieved from the Internet: <URL:http://medical-dictionary.thefreedicti onary.com/skin+lesions>

## Description

### Field of the invention

This invention concerns the use of a liquid composition, capable of promoting the growth of tissue cells at the site of a wound, a process that promotes regeneration of tissue and wound healing. More particularly, the present invention relates to the use of a liquid composition comprising a component (a) selected from the salts consisting of cations Aₙ⁺ and anions derived from halogen oxides according to the general formula [OₘX]⁻, a component (b) selected from the group of oxygen donors, and a component (d) selected from the group of liquid binders for the treatment of open wounds and burns.

### Background of the invention

The process of healing of open wounds and burns is a very complex, not fully understood process. Nowadays the most common means of treating open wounds or burns mainly comprise temporary wound closure/covering for protection against e.g. moisture loss and infection using autografts, dermal replacement products or hydrogels such as alginates, administration of substances for cooling and pain relief, and promoting wound healing by administering drugs which are usually hemostatic for suppressing bleeding in the wound region, anti-inflammatory for suppressing inflammation, sterilizers for sterilisation so as to prevent miscellaneous bacteria from invading the wound, or drugs with a combination of the above pharmaceutical effects. However, at present "reconstruction of tissue" as the final stage of wound healing has to rely on the auto-therapy inherent in living bodies.

Open wounds or burns that have impaired blood flow are often characterised by a delay or complete failure of the healing process. This is ascribed to the fact that in hypoxic tissue resistance to infection is lower and the fact that the process of auto-therapy is compromised. Wound oxygenation therefore has long been known to be beneficial in the healing of (chronic) wounds and burns. The healing of wounds and the effect of oxygen thereon has been intensively studied, a useful summary is presented in J.D. Whitney, "Physiological Effects of Tissue Oxygenation on Wound Healing", HEART & LUNG, september 1989, Vol. 18 No. 5, pp. 466-474.

Supplying an environment, rich in molecular oxygen, to the open wounds or burns, is believed to be beneficial in wound healing by activating the cells present at the periphery of wound sites by promoting their metabolism and by stimulating phagocytosis and killing of bacteria by neutrophils or polymorphonuclear cells (PMNs), which involves the production of oxygen radicals and superoxides and is directly influenced by the oxygen concentration in the tissue.

Active oxygen is quite different in view of chemical species and biochemical activities from ordinary molecular oxygen. While ordinary oxygen contributes only to metabolism but does not function as bio-signals for the growth of the cells, the active oxygen does not contribute to the metabolism but function as bio-signals for the growth of the cells.

In auto-therapy, the reconstruction of the tissues at the wound site is conducted through the following processes: (1) macrophages gathering at the wound site yield growth factors and enzymes, (2) the growth factors and the enzymes are activated, (3) the activated growth factors and enzymes stimulate increase or movement of fibroblasts to grow the cells, and (4) the tissue is reconstructed (regenerated) by the grown new cells.

In the case of auto-therapy, macrophages gathering at the wound sites produce active oxygen and the active oxygen functions as bio-signals to activate the growth factors and the enzymes yielded also from the macrophage. That is, it is known that active oxygen contributes to activation of growth factors and enzymes in the process of auto-therapy. Active oxygen supplied from the outside activates the growth factors and the enzymes together with active oxygen produced spontaneously from macrophage and promotes the growth of cells by a process similar to that of auto-therapy. As a result, growth of the cells is further promoted and the tissues at the wound site are reconstructed (regenerated) in a shorter period of time.

The importance of both ordinary molecular oxygen and of activated oxygen in these processes has once more been demonstrated by Cho et al. (Am. J. Physiol. Heart Circ. Physiol., 280; 2001), particularly in the process of angiogenesis. Their findings suggest that activated oxygen stimulates macrophages to release higher levels of VEGF (vascular endothelial growth factor) and can thereby drive angiogenesis in wounds. They found that neutrophils release higher amounts of activated oxygen when exposed to hyperoxia in vitro, and that VEGF itself is stimulated by hyperoxia in wound cylinders.

A plurality of methods for supplying either normal molecular oxygen or active oxygen to open wounds or burns is generally known in the art.

US 5.865.722 discloses a "topical" hyperbaric oxygen chamber which is a special type of the long known hyperbaric oxygen chamber. Hyperbaric oxygen chambers are for the purpose of introducing of pressurized molecular oxygen into an encapsulated environment with this oxygen to promote the healing of various types of wounds. Specifically it was mentioned that the treatment of open wounds within a hyperbaric oxygen chamber promotes healing and suppresses bacterial infection. "Topical" hyperbaric oxygen chambers have been proposed due to the expenses of large hyperbaric oxygen chambers and due to the risks of systemic oxygen toxicity thereof. These topical hyperbaric oxygen chambers, however, have the disadvantage of difficulties in air-tight sealing to the body without interfering with blood supply, resulting in operation under modestly elevated pressure, while consuming large amounts of oxygen, because of leakage.

US 5.792.090 discloses a more convenient means of increasing the wound oxygen tension, through the application of an oxygen generating wound dressing which renewably and non-sustainingly chemically generates oxygen. The wound dressing contains an aqueous liquid, containing hydrogen peroxide, in an oxygen supply solution reservoir, which also contains a solid hydrogen peroxide decomposition catalyst, such as manganese dioxide, the system thus being capable of supplying molecular oxygen through chemical reaction. US 6.139.876 discloses a gel such as a gelatine that can be saturated with oxygen, basically by heating it until the gelatine is a liquid, and introducing, in a closed vessel, oxygen gas into it so that the pressure is at least about 0.15 Mpa and mixing the liquid with oxygen; cooling it to enable a gelatine to form having excess oxygen therein in the form of microscopic bubbles. This oxygen containing gelatine can be applied to open wounds and burns for supplying molecular oxygen to said wound or burn. The major disadvantage of this method is that the gelatine has to be stored in a special container, wherein the pressure is maintained above atmospheric pressure during storage.

According to US 2002/0160053 A1 application of a solution containing activated oxygen can promote growth of new tissue cells at wound sites, by enhancing certain biochemical reactions in and around the region of the wound, by activation of the growth factors and enzymes, similar to the process of auto-therapy.

The invention, as disclosed in US 2002/0160053 A1, allows the aforementioned object to be achieved by producing an aqueous solution, which comprises water containing active oxygen and halogen ions. The active oxygen can include singlet oxygen (¹O₂) formed by excitation of triplet oxygen, superoxide (O₂⁻) formed by reduction of oxygen by a single electron, and hydroxy radical (HO^{●}), as well as hypochlorous ions (ClO⁻) and peroxy radicals (ROO^{●}), alkoxy radicals (RO^{●}), and hydroperoxides (ROOH).

A major drawback of the invention, according to US 2002/0160053 A1, is the fact that the solution, containing activated oxygen, has to be obtained by electrolysis, which is to be performed, according to the inventors, by the hospitals themselves, which is inconvenient and moreover suggests that these solutions are not very stable. Also, trained personnel is required to operate the electrolysis apparatus.

US 4.507.285 discloses aqueous compositions containing stabilised activated oxygen in a matrix of chlorite ions and pharmaceutical compositions, which may be applied for the treatment of skin diseases or for healing wounds and burns. The stabilised activated oxygen is present in the form of an aqueous solution. The production of the composition according to this invention involves many long (4 weeks!) and laborious reaction steps, which is clearly an inconvenient and costly process.

US 6.488.965 discloses a liquid preparation that is said to be suitable for the treatment of wounds and burns, said preparation comprising a metal chlorite and a peroxide compound, preferably hydrogen peroxide. The preparation may be in the form of a gel, cream or ointment. Successful treatments of wounds and burns are, however, not disclosed.

Document WO 00/19981 discloses the treatment of dermal ulcerations, in particular pressure ulcers (decubitus ulcers), venous ulcers, arterial ulcers and diabetic ulcers with a preparation comprising 0.001 to 0.20 % by weight of a metal chlorite and 0.001 to 0.05 % by weight of a peroxy compound. The preparation may be a solution, a gel, an ointment, a cream or a spray. The therapeutic effect is a synergy between the peroxide and the metal chlorite. There is thus still a need for a medicament for treatment of open wounds according to the claims. capable of promoting wound healing through the mechanism of auto-therapy, most preferably by supplying oxygen to the wound, either ordinary molecular oxygen or activated oxygen, which is convenient to use and easily and cost-effectively stored and produced.

US 6.017.515 discloses a combination preparation for bleaching teeth or treating skin complaints and mucous membrane disorders, especially lesions, said combination preparation being able, when mixed and applied topically, to deliver chemically generated oxygen. The composition according to US 6,017,515 comprises a component (a) selected from the salts consisting of cations Aₙ⁺ and anions derived from halogen oxides according to the general formula [OₘX]⁻, and a component (b) selected from the the salts consisting of cations Aₙ⁺ and anions derived from borates according to the general formula [BₚO_{q}]^{r-}, wherein A is a metal selected from Groups 1 or 2 of the Periodic System of the Elements, X is a halogen atom, m = 1 - 4, n = 1 or 2, p = 1 - 4, q = 1 - 8, and r = 1 - 3.

### Summary of the invention

We have surprisingly found that the active components from the invention disclosed in US 6.017.515 can be used successfully for promoting the healing of open wounds and burns by, without being bound to theory, chemically releasing oxygen. A composition containing these components can be used for the manufacture of a medicament that, when applied topically, promotes the healing of open wounds being selected from the group consisting of diabetic wounds, pressure ulcers, arterial ulcers, debuticus ulcers and venus ulcers. In a preferred embodiment of the present invention this composition can be provided with an oxygen donor stabilising agent.

### Detailed description of the invention

One aspect of the present invention relates to the use of a liquid composition as defined in the claims for the treatment of open wounds being selected from the group consisting of diabetic wounds, pressure ulcers, arterial ulcers, debuticus ulcers and venus ulcers, wherein the composition comprises a component (a) selected from the salts consisting of cations Aⁿ⁺ and anions derived from the halogen oxides according to the general formula [OₘX]⁻, wherein A is a metal selected from Groups 1 and 2 of the Periodic System of the Elements, X is a halogen atom, m = 1 - 4, n is 1 or 2, and a component (b) selected from the group of oxygen donors, and a component (d) selected from the group of liquid binders.. According to the present invention, the liquid composition is obtainable by combining at least components (a), (b) and (d).

In the formula [OₘX]⁻ X can be fluorine, chlorine, bromine or iodine, preferably chlorine. Examples of the anion [OₘX]⁻ are the hypochlorite, hypoiodite, chlorite, iodite, chlorate, bromate, iodate, perchlorate and periodate anions. According to the present invention it is preferred that m = 1, so that the anion [OₘX]⁻ is most preferably the hypochlorite anion.

Component (b) is selected from oxygen donors selected from the group consisting of metal perborate compounds (also known as peroxoborate compounds).

According to the invention, the halogen oxide compounds are preferably formed in situ from halogen oxide compound precursors. Suitable halogen oxide compound precursors are for example metal halite such as sodium chlorite and metal hypohalite such as sodium hypochlorite compounds as is well known in the art.

According to the invention, the perborate compounds are selected from the salts consisting of cations Aⁿ⁺ and anions derived from borates according to the general formula [BₚO_{q}]^{r-}, wherein A is a metal selected from Groups 1 or 2 of the Periodic System of the Elements, and p = 1 - 4, q = 1 - 8, and r = 1 - 3. Examples of the anion [BₚO_{q}]^{r-} are perborate (BO₃)⁻, metaborate (BO₂)⁻, orthoborate (BO₃)³⁻, hypoborate (B₂O₄)²⁻ and pyroborate or tetraborate anions (B₄O₇)²⁻. Preferably, p = 1, q = 2 or 3 and r = 1. Most preferably, p = 1, q = 3 and r = 1 which implies that the most preferred anion is perborate.

According to the invention, A is selected from the group consisting of lithium, sodium, potassium and calcium and is most preferably sodium. Consequently, component (a) is most preferably sodium hypochlorite and component (b) is most preferably sodium perborate.

Components (a) and (b) can contain one or more molecules of water as water of crystallisation. However, for component (a) preferably an aqueous solution thereof is used. Component (b) can also contain one or more molecules of water as water of crystallisation, for example the monohydrates, dihydrates, trihydrates and tetrahydrates. According to the invention, all hydrates of both components (a) and component (b) can be used. If component (a) or component (b) occur in different polymorphs, all these polymorphs can be used in the present invention.

For the sake of clarity, if component (b) is for example sodium perborate tetrahydrate, the compound Na₂BO₃.4H₂0 is intended which at present is more often defined as Na₂[B₂(O₂)₂(OH)₄].6H₂O; the common name of the later compound is sodium peroxoborate hexahydrate. Likewise, if component (b) is for example sodium perborate monohydrate, the compound Na₂BO₃.H₂0 is intended which at present is more often defined as Na₂[B₂(O₂)₂(OH)₄]. Reference is made to Kirk-Othmer, Encyclopedia of Chemical Technology, 4th Edition, Volume 18, pages 202 - 229 (1996) for the nomenclature of such compounds.

The present invention relates to the manufacture of a medicament, using a liquid composition comprising a component (a) and a component (b) as defined above. The term "liquid", as used herein, refers to any non-solid pharmaceutical formulation known in the art, which is suitable for topical application. In particular the present invention relates to a gel, cream, paste or ointment or a more liquid material such as a suspension, dispersion or emulsion.

The composition is used for the manufacture of a medicament, which can be any type of medicament for topical application known in the art. Particularly preferred medicaments, according to the present invention, are a wound gel, a wound spray, or a wound dressing. The use of component (a) or component (b) in the manufacture of any medicament, which is intended to be used for treatment of open wounds being selected from the group consisting of diabetic wounds, pressure ulcers, arterial ulcers, debuticus ulcers and venus ulcers by combined application of component (a) and component (b) falls within the scope of the present invention.

The term "wound dressing", as used herein, particularly refers to any material applied to a wound for protection, absorbance, drainage, etc. Numerous types of dressings are commercially available, including films (e.g., polyurethane films), hydrocolloids (hydrophilic colloidal particles bound to polyurethane foam), hydrogels (cross-linked polymers containing about at least 60% water), foams (hydrophilic or hydrophobic), calcium alginates (nonwoven composites of fibers from calcium alginate), and cellophane (cellulose with a plasticizer). According to one embodiment of the present invention, the medicament is such a wound dressing, which is impregnated or coated with the liquid, wound healing composition of the present invention.

In a particularly preferred embodiment a wound dressing is used that disintegrates and/or dissolves during contact with the wound or burn. In such an embodiment the dressing, which is impregnated or coated with the wound healing composition, is composed of a water-soluble polymer. Examples of such polymers that can be suitably used in the present invention are poly(lactic acid), poly(acrylic acid), and polyvinyl pyrrolidone.

The present invention relates to the manufacture of a medicament, using a composition, which comprises a component (a), a component (b) and a component (d), which medicament is able to supply an amount of chemically generated oxygen to the open wound or burn, which is effective in promoting wound healing. Said medicament comprises from 0.5 to 25% by weight of the total composition of component (a), more preferably from about 1.0 % to about 20.0 % by weight. The medicament of the present invention comprises from about 0.1 % to about 8.0 % by weight of the total composition of component (b), more preferably from about 0.2 % to about 6.0 %, most preferably from about 0.3 % to about 3.0 % by weight, calculated on the total weight of the composition. The medicament further comprises from about 1.0 % to about 80.0 % by weight of the total composition of component (d), preferably from about 2.0 % to about 50.0 %, most preferably from about 3.0 % to about 20.0 % by weight.

The liquid binder according to the invention is used in particular for dispersing the components (a) and (b) and optionally (c), as explained below, and for enhancing the stability of the composition. Moreover, the liquid binder is used to adjust the concentrations of the active ingredients of the composition according to the invention. Obviously, the liquid binder has also additional properties, e.g. thickening properties, stabilising properties, water-binding promoting properties as is well known to the person skilled in the art. These liquid binders are selected from the liquid polyols, polymeric binders, fumed silica and gums or a combination thereof. Examples of suitable liquid polyols include glycerol, propylene glycol, polyethylene glycol (PEG). Examples of suitable gums include natural gums and modified (semi-synthetic) gums, for example acacia gum, gum arabic, caraya gum, gum tragacanth, xanthan gum and cellulose gum. Examples of suitable polymeric binders are polyvinyl pyrrolidone, casein or salts thereof, wheren the salts comprise a metal of Group 1 or Group 2 of the Periodic System. According to the invention, it is preferred that the liquid binder is glycerol, glycol, propylene glycol, PEG, fumed silica, a gum, or a combination thereof. In a particularly preferred embodiment of the present invention the liquid binder is a combination of a liquid polyol and a fumed silica, most preferably PEG 1500 in combination with fumed silica, in total amounts of 0.005 to 4 % and 1 to 20 %, respectively, based on the total weight of the composition. Most preferred is an amount of PEG 1500 from about 0.01 % to about 2 % by weight of the total composition, and an amount of fumed silica from about 3 % to about 10 % by weight of the total composition. Moreover, component (a) is preferably employed as an aqueous solution comprising the binder, said aqueous solution comprising 25 - 75 % by weight, preferably 35 - 65 % by weight of the binder, calculated on the basis of the total weight of the aqueous solution.

Additionally, according to the invention the pH of the composition is essential for a controlled and long lasting release of the active component, i.e. oxygen. Tests have revealed that the pH is preferably in the range of 4 - 8, preferably 4,5 - 7,5 and most preferably 5,0 - 7,5.

The composition according to the present invention, which is used for the treatment of open wounds and burns, may further comprise a gelatinous thickener. Typically a cellulose material, such as cellulose, sodium carboxymethylcellulose, (hydroxy)propylcellulose, methylcellulose, or ethylcellulose, is used as a thickener. Preferably sodium carboxymethylcellulose is used in the present invention, in an amount of 0.2 to 4.0 percent by weight, preferably 0.5 to 2.5 percent by weight, calculated on the total weight of the composition.

The composition may further comprise an agent that counteracts loss of moisture, and that optionally also has an anti-microbial action. Preferably a carbohydrate, more preferably an alditol, such as, for example, erythritol, arabinitol, xylitol, galacitol, sorbitol, iditol, mannitol, hepitol, or octitol, is used as the agent that counteracts the loss of moisture. In the present invention the use of alditol is preferred, typically in an amount of 0.5 to 10.0 percent by weight preferably in an amount of 1.0 to 5.0 percent by weight, calculated on the total weight of the composition.

Preferably the compositions according to the present invention contain an anti-oxidant. Examples of suitable anti-oxidants are Lipochroman-6, sodium ascorbylphosphate, or combinations thereof. Preferably the compositions contain an amount of anti-oxidant of about 0.10 % to about 4.0 % by weight of the total composition. In a preferred embodiment Lipochroman-6 and sodium ascorbylphosphate are used. Preferably the compositions contain from about 0.01 % to about 1.0 % by weight of Lipochroman-6, and from about 0.10 % to about 3.00 % by weight of sodium ascorbylphosphate.

The compositions and medicaments according to the present invention preferably comprise a components selected from the group of sulfa drugs that are used to treat bacterial and some fungal infections. Suitable sulfa drugs comprise prontosil, sulfadiazine, sulfamethizole (Thiosulfil Forte®), sulfamethoxazole (Gantanol®), sulfasalazine (Azulfidine®), sulfisoxazole (Gantrisin®), and various high-strength combinations of three sulfonamides. Preferably, the sulfa drug is sulfadiazine.

The compositions and medicaments according to the present invention further preferably comprise a zinc component which are beneficial in wound healing. A suitable example is zinc gluconate.

The compositions and medicaments according to the present invention further preferably comprise an agent that promotes degradation of biofilms on open wounds. Suitbale agents include peroxide forming enzymes such as lactoperoxidase as is disclosed in WO 88/02600 of Poulson and glycoproteins such as lactoferrin as disclosed in EP A 1.545.5 87 .

The compositions according to the present invention can optionally further comprise any pharmaceutically acceptable excipient, such as, for example, colorants, (de)odorants, preservatives and the like. The composition, according to the present invention, is intended for use in the treatment of open wounds. The term "open wound", as used herein, refers to tissue injuries characterised by delay or complete failure of healing. Such injuries are resulting from diabetic wounds, pressure ulcers, arterial ulcers, decubicus ulcers and venous ulcers. The greatest benefits are achieved in injured tissues with compromised blood flow and oxygen supply.

The treatment of open wounds according to the present invention typically comprises topical administration of the medicament or of a combination of the medicaments, containing the composition, to the open wound. The medicament is preferably applied to the wounds or burns in amounts sufficient to completely cover the entire surface of the wound. In a preferred embodiment, the composition is applied to the open wound or burn, 1 to 8 times daily, more preferably 2 to 4 times daily. The treatment is continued as long as necessary to completely heal the wounds, it is applied to, or as long as beneficial effects are observed.

Although the aforementioned method of treatment generally applies, it is within the skill and within the objective of any professional, trained in the art of wound healing, to adjust the preferred amounts of the medicament and/or the frequency it is applied with, as well as the duration of the treatment, in order to optimise the efficacy for each individual patient.

In another embodiment of the present invention the composition further comprises an oxygen donor stabilising agent (component (c)). Addition of an oxygen donor stabilising agent will result in a composition, which releases the active component in a more controlled manner and which shows a longer lasting effect. Compositions containing the oxygen stabilising agent will further have improved stability during storage and transport under normal conditions. Preferably the oxygen donor stabilising agent is selected from the group consisting of organic acids or their (monovalent or polyvalent) pharmaceutically acceptable salts, preferably inorganic salts wherein the cations of the salts are preferably metals selected from groups 1 or 2 of the Periodic System of the Elements, or from the group consisting of saccharides.

The organic acids are preferably selected from the group consisting of chelating organic acids. The chelating organic acids are preferably selected from carboxylic acids containing one or more hydroxy and/or amino groups or from polycarboxylic acids optionally containing one or more hydroxy and/or amino groups.

Alternatively but depending on the intended use of the composition according to the invention, the chelating organic acids may be selected from polyphosphonic acids or their pharmaceutically acceptable salts as disclosed in US 6.265.444 which is herein incorporated by reference, although then it is preferred that the polyphosphonic acid is used in combination with a carboxylic acid containing one or more hydroxy and/or amino groups or polycarboxylic acid optionally containing one or more hydroxy and/or amino groups such as EDTA. In this patent application a polyphosphonic acid is to be understood as a compound containing at least two -PO₃H₂ moieties or the pharmaceutical acceptable salt thereof. Preferably, the polyphosphonic acid has either formula I or formula II, 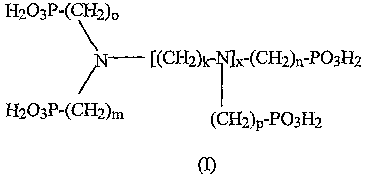 wherein x is an integer of 0 to 3 and k, m, n, o and p are each independently an integer of 1 to 4. Preferably, x is 2 and k, m, n, o and p are each 1 or 2; 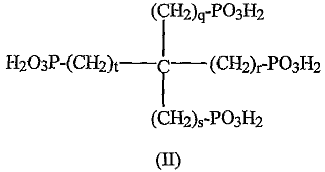 wherein q, r, s and t are each independently an integer of 0 to 4. Preferably q, r, s and t are 0 or 1, and most preferably all are zero.

According to the invention, it is most preferred that the polycarboxylic acids contain one or more hydroxy and/or amino groups and such acids are generally known as hydroxypolycarboxylic acids and aminopolycarboxylic acids. Very suitable examples of the oxygen donor stabilising agents according to the invention are gluconic acid, ethylenediaminotetraacetic acid (EDTA), nitrilotriacetic acid (or 1,1',1"-tricarboxytrimethylamine) and methylglycinediacetic acid or their salts. Most particular the oxygen donor stabilising agent is sodium gluconate.

The saccharides are preferably monosaccharides or disaccharides. Preferred examples are glucose, fructose, sucrose, maltitol and lactitol.

According to the invention, the molar ratio of component (b) to the oxygen donor stabilising agent is preferably 0.1 - 5.0 (b) : 1.0 oxygen donor stabilising agent. More preferably, this molar ratio is 0.1 - 5.0 (b) : 1.0 oxygen donor stabilising agent and in particular 0.1 - 5.0 (b) : 1.0 oxygen donor stabilising agent

Accordingly the molar ratio of component (a) to the oxygen donor stabilising agent is preferably 1.0-30.0 (a) : 1.0 oxygen donor stabilising agent. More preferably, this molar ratio is 5.0-25.0 (a) : 1.0 oxygen donor stabilising agent and in particular 10.0-20.0 (a) : 1.0 oxygen donor stabilising agent.

In the preferred embodiment of the present invention where the composition comprises the oxygen donor stabilising agent, the composition is used for the treatment of open wounds and burns, wherein the method comprises topically administering an amount of the medicament or of a combination of the medicaments, containing the composition, to the open wound or burn. The medicament is applied to the wounds or burns in amounts that are sufficient to completely cover the entire surface of the wound. In a preferred embodiment, the composition is applied to the open wound or burn, 1 to 8 times daily, more preferably 2 to 4 times daily. The treatment is continued as long as necessary to completely heal the wounds, it is applied to, or as long as beneficial effects are observed.

The medicament according to the present invention may be used in combination with any other known method or means for treating wounds and burns, as can be determined by any skilled professional in the art.

The invention is further illustrated by the following examples which are not intended to limit the scope of protection conferred by the claims.

### EXAMPLE 1

A wound gel and a wound spray were prepared according to the present invention, which can be used for the treatment of open wounds and burns by topical application. The compositions of the wound gel and wound spray are shown in table 1.

**Table 1**

| | wound gel | wound spray |
|---|---|---|
| water | 51.5 % | 56.5 % |
| Comp. (a) | 2.5 % | 2.5 % |
| Comp (b) | 1.72 % | 1.72 % |
| Comp. (c) | 1.0 % | 1.0 % |
| Wacker HDK20 (liquid binder) | 5.00 % | 0 % |
| PEG 1500 (liquid binder) | 0.1 % | 0 % |
| sucrose | 2.0 % | 2.0 % |
| ethanol (99.9 %) | 20.0 % | 20.0 % |
| glycerine | 15.000 % | 15.000 % |
| cellulose gum | 0.6 % | 0.600 % |
| citric acid.H₂O | 0.6 % | 0.63 % |

### EXAMPLE 2

In this experiment, an open wound on the lateral side of the left lower leg, which had arisen spontaneously in a diabetic patient, was treated by chemical sympathectomy and by topical application of wound gel from Example 1, following a dosing scheme that was assessed, and adjusted when deemed necessary by a skilled healthcare professional. It is generally known that the healing process of diabetic ulcers can last up to several months with the currently known treatments, and even up to longer periods without treatment. After two weeks of treatment the wound was re-examined and an apparent improvement was seen; the wound surface had become cleaner. The treatment with the wound gel of the present invention was continued. In the following weeks the appearance of the wound became cleaner and after 14 weeks of treatment with the wound gel an apparent decrease in wound diameter became observable. The wound diameter had decreased substantially when re-examined after 6 months of treatment. The treatment with the wound gel was continued. During treatment the patient was re-examined several times and during each examination the wound was photographed in the exact same position each time, so as to obtain a series of photographs of the wound during the whole period of treatment that could be used to calculate surface of the wound (expressed in the amount of pixels that were comprised by the wound on each photograph) and wound size reduction. In table 2 the healing process is represented as the amount of pixels comprised by the wound on each photograph, the reduction of the size of the wound surface (amount of pixels), and the relative size of the wound on each moment, as a percentage of the initial size of the wound surface.

**Table 2**

| examination | wound surface (pixels) | decrease of wound surface compared to day 0 (pixels) | percentage of the wound surface on day 0 |
|---|---|---|---|
| day 0 | 85,608 | 0 | 100 |
| day 14 | 82,720 | 2,888 | 96,62648 |
| day 70 | 93,184 | -7,576 | 108,8496 |
| day 97 | 81,295 | 4,313 | 94,96192 |
| day 136 | 81,184 | 4,424 | 94,83226 |
| day 188 | 66,792 | 18,816 | 78,02075 |
| day 259 | 28,314 | 57,294 | 33,07401 |

### EXAMPLE 3

An injury in the lower leg of a patient, which had become necrotic and contained haematoma, could not be treated with flammazine (Merck Index 13th Ed., page 1586, nr. 8988 (2001)), which is at present one of the common medicaments for treating wounds such as ulcurs and burns, because of an allergy. Therefore it was treated with the wound gel, according to Example 1, by filling the wound with the gel twice daily. After two weeks of treatment according to this regimen, re-examination revealed that the wound surface had decreased significantly and had a clean, normal appearance. Quantitative evaluation of photographs of the wound at day 0 and day 14, revealed that the wound surface had decreased with 76 %, within the two weeks of treatment. It is generally known that the healing of necrotic injuries, with haematoma, can last up to several motnhs when treated with the presently common methods.

### EXAMPLE 4

A group of seven patients suffering from chronic and/or slow-healing wounds from various causes were treated with a composition according to the present invention. The composition was applied as a gel or a spray, according to example 1, following a regimen as encompassed by the present invention, tailor made to meet the specific needs and requirements of each individual case, as assessed by professional healthcare personnel. During the period of treatment the wounds were re-examined periodically. During each examination the wounds were photographed in the exact same position each time, so as to obtain a series of photographs of each wound that could be used to calculate surface of the wound (expressed in the amount of pixels that were comprised by the wound on each photograph) and wound size reduction. The results of these experiments are summarized in Table 3.

### EXAMPLE 5

A spina bifida patient that had been suffering from an open wound on the heel of the right foot for more than six months, entered the present study. The subject had been treated with flammazine, which is presently one of the common methods of treating such wounds. Initially this had resulted in a noticeable decrease of the surface of the wound and an improving appearance. However, after several weeks the healing process of the wound ceased with this treatment. On entering the study, the patient was switched to a combined regimen of a wound gel according to the present invention and a flammazine ointment, applied topically to the ulcur on the heel of the right foot twice daily in such amounts that it completely covered the entire surface of the wound. After 114 days of treatment the wound had completely closed.

**Table 3**

| patient | description of the wound | treatment | initial wound surface (pixels) | wound surface (pixels) after treatment | difference (pixels) | % reduction |
|---|---|---|---|---|---|---|
| 1 | chronic venous insufficiency right foot/ankle, lateral side, diabetic patient | gel, 196 days | 9,702 | closed | 9,702 | 100 % |
| 2 | ulcus with necrosis, left leg, lateral side | gel, 260 days | 19,300 | closed | 19,300 | 100 % |
| 3 | decubitus on the left heel | spray, 182 days | 31,390 | 22,477 | 21,562 | 68.7 % |
| 4 | chronic ulcer, on the right lower leg, resulting from trauma | gel, 126 days | 28,728 | 22,477 | 6,251 | 21.8 % |
| 5 | chronic wound with necrosis, on the left leg, resulting from surgery | gel, 133 days | 42,746 | 35,179 | 28,326 | 66.3 % |
| 6 | chronic venous ulcer on the left lower leg with sclerosis | gel, 126 days | 50,702 | closed | 50,702 | 100 % |
| 7 | defect of the forefoot after amputation, diabetic patient | gel, 331 days | 37,742 | 15,510 | 22,232 | 58.9 % |

## Claims

1. Use of a liquid composition for the manufacture of a medicament for the treatment of open wounds, the open wound being selected from the group consisting of diabetic wounds, pressure ulcers, arterial ulcers, debuticus ulcers and venous stasis ulcers, said composition comprising:
(a) 0.5 to 25 wt.%, calculated on the total weight of the composition, of a component (a) selected from the salts consisting of cations Aⁿ⁺ and anions derived from halogen oxides according to the general formula [OₘX]⁻, wherein A is a metal selected from Groups 1 or 2 of the Periodic System of the Elements and is lithium, sodium, potassium or calcium, X is a halogen atom, m = 1 - 4, n is 1 or 2;
(b) 0.1 to 8 wt.%, calculated on the total weight of the composition, of a component (b) selected from the group of oxygen donors, wherein the oxygen donor is a metal perborate compound which is selected from the salts consisting of cations Aⁿ⁺ and anions derived from borates according to the general formula [BₚO_{q}]^{r-}, wherein A is a metal selected from Groups 1 or 2 of the Periodic System of the Elements and is lithium, sodium, potassium or calcium, p = 1 - 4, q = 1 - 8 and r = 1 - 3; and
(c) a component (d) selected from the group of liquid binders, wherein the liquid binder is selected from the group consisting of liquid polyols, polymeric binders, fumed silica, gums and combinations thereof.

2. Use of a liquid composition according to Claim 1, said composition being obtainable by combining at least:
(a) a component (a) selected from the salts consisting of cations Aⁿ⁺ and anions derived from halogen oxides according to the general formula [OₘX]⁻, wherein A is a metal selected from Groups 1 or 2 of the Periodic System of the Elements and is lithium, sodium, potassium or calcium, X is a halogen atom, m = 1 - 4, n is 1 or 2;
(b) a component (b) selected from the group of oxygen donors, wherein the oxygen donor is a metal perborate compound which is selected from the salts consisting of cations Aⁿ⁺ and anions derived from borates according to the general formula [BₚO_{q}]^{r-}, wherein A is a metal selected from Groups 1 or 2 of the Periodic System of the Elements and is lithium, sodium, potassium or calcium, p = 1 - 4, q = 1 - 8 and r = 1 - 3; and
(c) a component (d) selected from the groups of liquid binders, wherein the liquid binder is selected from the group consisting of liquid polyols, polymeric binders, fumed silica, gums and combinations thereof

3. Use according to Claim 1 or Claim 2, wherein A is lithium, sodium, potassium or calcium, n = m = p = 1, q = 2 or 3 and r = 1 - 3.

4. Use according to any one of Claims 1 - 3, wherein A is sodium, X is chlorine, p = 1, q = 3 and r = 1.

5. Use according to any one of Claims 1 - 4, wherein compound (a) is sodium hypochlorite.

6. Use according to any one of Claims 1 - 5, wherein compound (b) is sodium perborate.

7. Use according to any one of Claims 1 - 6, wherein the composition further comprises a gelatinous thickener.

8. Use according to Claim 7, wherein the gelatinous thickener is a cellulose material.

9. Use according to any one of Claims 1 - 8, wherein the composition further comprises an agent that counteracts loss of moisture, wherein the agent that counteracts loss of moisture is a carbohydrate.

10. Use according to any one of Claims 1 - 9, wherein the composition further comprises an anti-oxidant.

11. Use according to any one of Claims 1 - 10, wherein the medicament is topically applied to open wounds 2 - 4 times a day.

12. Use according to any one of Claims 1 - 11, wherein the composition further comprises a component (c) selected from oxygen donor stabilising agents.

13. Use according to Claim 12, wherein the oxygen donor stabilising agent is selected from the group consisting of organic acids and salts thereof, wherein the organic acid is selected from the group consisting of carboxylic acids containing one or more hydroxy and/or amino groups and polycarboxylic acids optionally containing one or more hydroxy and/or amino groups.

14. Use according to any one of Claims 1 - 13, wherein the medicament is a wound gel, a wound spray, or a wound dressing.

15. Wound dressing for use for the treatment of open wounds, the open wound being selected from the group consisting of diabetic wounds, pressure ulcers, arterial ulcers, debuticus ulcers and venous stasis ulcers, the wound dressing comprising the liquid composition as defined in any one of Claims 1 - 14.

## Patentansprüche

1. Verwendung einer flüssigen Zusammensetzung zur Herstellung eines Arzneimittels für die Behandlung offener Wunden, wobei die offene Wunde aus der Gruppe ausgewählt ist, die aus diabetischen Wunden, Druckgeschwüren, arteriellen Geschwüren, Dekubitalulcera und Venenstaugeschwüren besteht, und die Zusammensetzung:
a) 0,5 bis 25 Gew.-%, bezogen auf das Gesamtgewicht der Zusammensetzung, einer Komponente (a), die aus den Salzen ausgewählt ist, die aus Kationen Aⁿ⁺ und Anionen, die von Halogenoxiden mit der allgemeinen Formel [OₘX]⁻ abgeleitet sind, bestehen, wobei A ein Metall, das aus der Gruppe 1 oder 2 des Periodensystems der Elemente ausgewählt ist und Lithium, Natrium, Kalium oder Calcium ist, X ein Halogenatom ist, m = 1 bis 4 und
n 1 oder 2 ist,
b) 0,1 bis 8 Gew.-%, bezogen auf das Gesamtgewicht der Zusammensetzung, einer Komponente (b), die aus einer Gruppe von Sauerstoffdonoren ausgewählt ist, wobei der Sauerstoffdonor eine Metallperboratverbindung ist, die aus den Salzen ausgewählt ist, die aus Kationen Aⁿ⁺ und Anionen, die von Boraten mit der allgemeinen Formel [BₚO_{q}]^{r-} abgeleitet sind, bestehen, wobei A ein Metall, das aus der Gruppe 1 oder 2 des Periodensystems der Elemente ausgewählt ist und Lithium, Natrium, Kalium oder Calcium ist, p = 1 bis 4, q = 1 bis 8 und r = 1 bis 3 ist, und
c) eine Komponente (d), die aus einer Gruppe ausgewählt ist, die aus flüssigen Bindemitteln besteht, wobei das flüssige Bindemittel aus einer Gruppe ausgewählt ist, die aus flüssigen Polyolen, polymeren Bindemitteln, pyrogener Kieselsäure, Gummen und Kombinationen davon besteht,
umfasst.

2. Verwendung einer flüssigen Zusammensetzung nach Anspruch 1, wobei die Zusammensetzung durch Kombination von wenigstens:
a) einer Komponente (a), die aus den Salzen ausgewählt ist, die aus Kationen Aⁿ⁺ und Anionen, die von Halogenoxiden mit der allgemeinen Formel [OₘX]⁻ abgeleitet sind, bestehen, wobei A ein Metall, das aus der Gruppe 1 oder 2 des Periodensystems der Elemente ausgewählt ist und Lithium, Natrium, Kalium oder Calcium ist, X ein Halogenatom ist, m = 1 bis 4 und
n = 1 oder 2 ist,
b) einer Komponente (b), die aus einer Gruppe von Sauerstoffdonoren ausgewählt ist, wobei der Sauerstoffdonor eine Metallperboratverbindung ist, die aus den Salzen ausgewählt ist, die aus Kationen Aⁿ⁺ und Anionen, die von Boraten mit der allgemeinen Formel [BₚO_{q}]^{r-} abgeleitet sind, bestehen, wobei A ein Metall, das aus der Gruppe 1 oder 2 des Periodensystems der Elemente ausgewählt ist und Lithium, Natrium, Kalium oder Calcium ist, p = 1 bis 4, q = 1 bis 8 und r = 1 bis 3 ist, und
c) einer Komponente (d), die aus einer Gruppe flüssiger Bindemittel ausgewählt ist, wobei das flüssige Bindemittel aus einer Gruppe ausgewählt ist, die aus flüssigen Polyolen, polymeren Bindemitteln, pyrogener Kieselsäure, Gummen und Kombinationen davon besteht,
erhältlich ist.

3. Verwendung nach Anspruch 1 oder 2, wobei A Lithium, Natrium, Kalium oder Calcium ist, n = m = p = 1, q = 2 oder 3 und r = 1 bis 3 ist.

4. Verwendung nach einem der Ansprüche 1 bis 3, wobei A Natrium, X Chlor, p = 1, q = 3 und r = 1 ist.

5. Verwendung nach einem der Ansprüche 1 bis 4, wobei die Verbindung (a) Natriumhypochlorit ist.

6. Verwendung nach einem der Ansprüche 1 bis 5, wobei die Verbindung (b) Natriumperborat ist.

7. Verwendung nach einem der Ansprüche 1 bis 6, wobei die Zusammensetzung ferner ein gelatineartiges Verdickungsmittel umfasst.

8. Verwendung nach Anspruch 7, wobei das gelatineartige Verdickungsmittel ein Cellulosematerial ist.

9. Verwendung nach einem der Ansprüche 1 bis 8, wobei die Zusammensetzung ferner ein Mittel, das Feuchtigkeitsverlust entgegenwirkt umfasst, wobei das Mittel, das Feuchtigkeitsverlust entgegenwirkt ein Kohlenhydrat ist.

10. Verwendung nach einem der Ansprüche 1 bis 9, wobei die Zusammensetzung ferner ein Antioxidationsmittel umfasst.

11. Verwendung nach einem der Ansprüche 1 bis 10, wobei das Arzneimittel 2- bis 4-mal täglich topisch auf eine offene Wunde aufgetragen wird.

12. Verwendung nach einem der Ansprüche 1 bis 11, wobei die Zusammensetzung ferner eine Komponente (c), die aus Sauerstoffdonor-stabilisierenden Mitteln ausgewählt ist, umfasst.

13. Verwendung nach Anspruch 12, wobei das Sauerstoffdonor-stabilisierende Mittel aus der Gruppe ausgewählt ist, die aus organischen Säuren und Salzen davon besteht, wobei die organische Säure aus der Gruppe ausgewählt ist, die aus Carbonsäuren, die eine oder mehrere Hydroxy- und/oder Aminogruppen besitzen, und Polycarbonsäuren, die optional eine oder mehrere Hydroxy- und/oder Aminogruppen besitzen, besteht.

14. Verwendung nach einem der Ansprüche 1 bis 13, wobei das Arzneimittel ein Wundgel, ein Wundspray oder ein Wundverband ist.

15. Wundverband zur Verwendung für die Behandlung offener Wunden, wobei die offene Wunde aus der Gruppe ausgewählt ist, die aus diabetischen Wunden, Druckgeschwüren, arteriellen Geschwüren, Dekubitalulcera und Venenstaugeschwüren besteht, und der Wundverband die flüssige Zusammensetzung nach einem der Ansprüche 1 bis 14 umfasst.

## Revendications

1. Utilisation d'une composition liquide pour la production d'un médicament destiné au traitement des blessures ouvertes, la blessure ouverte appartenant au groupe comprenant les plaies diabétiques, les ulcères de pression, les ulcères artériels, les escarres et les ulcères veineux, ladite composition comprenant :
a) de 0,5 à 25 % en poids par rapport au poids total de la composition d'un composant (a) choisi parmi les sels constitués de cations Aⁿ⁺ et d'anions dérivés d'oxydes d'halogènes selon la formule générale [OₘX]⁻, dans laquelle A est un métal choisi dans les groupes 1 ou 2 du tableau périodique des éléments et est le lithium, le sodium, le potassium ou le calcium, X est un atome d'halogène, m = 1 - 4, n est égal à 1 ou 2 ;
b) de 0,1 à 8 % en poids, par rapport au poids total de la composition, d'un composant (b) choisi dans le groupe des donneurs d'oxygène, dans laquelle le donneur d'oxygène est un composé perborate métallique choisi parmi les sels constitués de cations Aⁿ⁺ et d'anions dérivés de borates selon la formule générale [BₚO_{q}]^{r-}, dans lequel A est un métal choisi dans les groupes 1 ou 2 du tableau périodique des éléments et est le lithium, le sodium, le potassium ou le calcium, p = 1 - 4, q = 1 - 8 et r = 1 - 3 ; et
c) un composant (d) choisi dans le groupe des liants liquides, dans laquelle le liant liquide est choisi dans le groupe comprenant les polyols liquides, les liants polymériques, la silice pyrogénée, les gommes et les combinaisons de ceux-ci.

2. Utilisation d'une composition liquide selon la revendication 1, ladite composition pouvant être obtenue en combinant au moins :
a) un composant (a) choisi parmi les sels constitués de cations Aⁿ⁺ et d'anions dérivés d'oxydes d'halogènes selon la formule générale [OₘX]⁻, dans laquelle A est un métal choisi dans les groupes 1 ou 2 du tableau périodique des éléments et est le lithium, le sodium, le potassium ou le calcium, X est un atome d'halogène, m = 1 - 4, n est égal à 1 ou 2 ;
b) un composant (b) choisi dans le groupe des donneurs d'oxygène, dans lequel le donneur d'oxygène est un composé perborate métallique choisi parmi les sels constitués de cations Aⁿ⁺ et d'anions dérivés de borates selon la formule générale [BₚO_{q}]^{r-}, dans laquelle A est un métal choisi dans les groupes 1 ou 2 du tableau périodique des éléments et est le lithium, le sodium, le potassium ou le calcium, p = 1 - 4, q = 1 - 8 et r = 1 - 3 ; et
c) un composant (d) choisi dans le groupe des liants liquides, dans lequel le liant liquide est choisi dans le groupe comprenant les polyols liquides, les liants polymériques, la silice pyrogénée, les gommes et les combinaisons de ceux-ci.

3. Utilisation selon la revendication 1 ou la revendication 2, dans laquelle A est le lithium, le sodium, le potassium ou le calcium, n = m = p = 1, q = 2 ou 3 et r = 1- 3.

4. Utilisation selon l'une quelconque des revendications 1 à 3, dans laquelle A est le sodium, X est le chlore, p = 1, q = 3 et r = 1.

5. Utilisation selon l'une quelconque des revendications 1 à 4, dans laquelle le composé (a) est l'hypochlorite de sodium.

6. Utilisation selon l'une quelconque des revendications 1 à 5, dans laquelle le composé (b) est le perborate de sodium.

7. Utilisation selon l'une quelconque des revendications 1 à 6, dans laquelle la composition comprend en outre un agent épaississant gélatineux.

8. Utilisation selon la revendication 7, dans laquelle l'agent épaississant gélatineux est un matériau cellulosique.

9. Utilisation selon l'une quelconque des revendications 1 à 8, dans laquelle la composition comprend en outre un agent destiné à prévenir le dessèchement, dans laquelle l'agent anti dessèchement est un carbohydrate.

10. Utilisation selon l'une quelconque des revendications 1 à 9, dans laquelle la composition comprend en outre un antioxydant.

11. Utilisation selon l'une quelconque des revendications 1 à 10, dans laquelle le médicament est appliqué localement sur les blessures ouvertes 2 à 4 fois par jour.

12. Utilisation selon l'une quelconque des revendications 1 à 11, dans laquelle la composition comprend en outre un composant (c) choisi parmi les agents stabilisateurs de donneurs d'oxygène.

13. Utilisation selon la revendication 12, dans laquelle l'agent stabilisateur de donneurs d'oxygène est choisi dans le groupe comprenant les acides organiques et leurs sels, dans laquelle l'acide organique est choisi dans le groupe comprenant les acides carboxyliques contenant un ou plusieurs groupes hydroxy et/ou amino et les acides polycarboxyliques contenant optionnellement un ou plusieurs groupes hydroxy et/ou amino.

14. Utilisation selon l'une quelconque des revendications 1 à 13, dans laquelle le médicament est un gel pour les plaies, un spray pour les plaies ou un pansement.

15. Pansement destiné à être utilisé pour le traitement des blessures ouvertes, la blessure ouverte étant choisie dans le groupe comprenant les plaies diabétiques, les ulcères de pression, les ulcères artériels, les escarres et les ulcères veineux, le pansement comprenant la composition liquide définie dans l'une quelconque des revendications 1 à 14.
